# EUROPÄISCHE PATENTSCHRIFT

(11) **EP 2 904 982 B1**
(45) Veröffentlichungstag und Bekanntmachung des Hinweises auf die Patenterteilung: **07.04.2021**
(21) Anmeldenummer: 14154490.8
(22) Anmeldetag: 10.02.2014
(51) Int. Cl.: A61B 18/12, H01R 13/518

(54) **Elektrochirurgisches Gerät mit einem Buchseneinsatz, Set mit einem Entnahmewerkzeug und Verfahren zum entnehmen eines Buchseneinsatzes**
Electrosurgical device having a female insert, set with a removing tool and method of removing a female insert
Appareil électrochirurgical doté d'un embout de douille, kit comprenant un outil de prélèvement et procéde de suppression d'un embout de douille

(43) Veröffentlichungstag der Anmeldung: 12.08.2015
(73) Patentinhaber: Erbe Elektromedizin GmbH, 72072 Tübingen (DE)
(72) Erfinder: Bopp, Benjamin, 72131 Ofterdingen (DE)
(74) Vertreter: Bohnenberger, Johannes

(56) Entgegenhaltungen:
- WO-A1-2009/018668
- DE-U1-202011 050 061
- US-A1- 2009 269 962
- US-A1- 2011 087 212

## Beschreibung

Die Erfindung betrifft ein elektrochirurgisches Gerät mit einem Buchseneinsatz sowie ein Set mit einem derartigen Buchseneinsatz und einem Entnahmewerkzeug. Die Erfindung betrifft ferner ein Verfahren zum Entnehmen eines solchen Buchseneinsatzes.

Aus der Praxis sind elektrochirurgische Geräte, beispielsweise HF-Chirurgiegeräte, bekannt, die mehrere Buchsen zum Anschluss von chirurgischen Instrumenten aufweisen. Die Buchsen bilden eine Steckverbindung zwischen den elektrochirurgischen Instrumenten und den Schaltkreisen im Inneren des elektrochirurgischen Geräts. Üblicherweise sind die Buchsen in Buchseneinsätze integriert, die fest im elektrochirurgischen Gerät, vorzugsweise an dessen Gehäuse, befestigt sind.

Im täglichen Gebrauch bestehen Anforderungen, einen Buchseneinsatz zu tauschen. Beispielsweise kann es vom Anwender gewünscht sein, ein elektrochirurgisches Instrument mit einem Stecker an ein Gerät anzuschließen, wobei der Stecker in die vorhandene Buchse nicht passt. Dies erfordert dann einen Austausch des Buchseneinsatzes. Zusätzlich werden die Steckverbindungen, die durch die Buchsen gebildet sind, häufig betätigt, so dass es zu einem hohen Verschleiß an den Buchsen kommt. Folglich kann es notwendig sein, die Buchsen oder Buchseneinsätze auszutauschen, um eine sichere Steckverbindung zu gewährleisten. Da die Buchsen bei bisher bekannten chirurgischen Geräten fest im Gerät verankert sind, ist zum Austausch der Buchsen ein Öffnen des Geräts erforderlich. Aufgrund gesetzlicher Bestimmungen darf ein derartiger Eingriff an dem Gerät ausschließlich durch geschultes Servicepersonal durchgeführt werden. Außerdem ist es erforderlich, nach dem Wiederverschließen des Gerätegehäuses eine umfangreiche sicherheitstechnische Kontrolle durchzuführen. Derartige sicherheitstechnische Kontrollen sind zeit- und kostenaufwändig.

DE 20 2011 050 061 U1 offenbart ein Kupplungssystem eines chirurgischen Geräts, das ein Handstück sowie eine Kupplungsvorrichtung aufweist. Die Kupplungsvorrichtung umfasst eine Arretiervorrichtung. Eine ähnliche Vorrichtung ist aus US 2009/0269962 A1 bekannt. Die aus dem Stand der Technik bekannten Gerätebuchsen sind allerdings nicht austauschbar, wodurch die Variabilität der chirurgischen Vorrichtung eingeschränkt ist.

Die Aufgabe der Erfindung besteht darin, ein elektrochirurgisches Gerät mit einem Buchseneinsatz anzugeben, der einen einfachen Austausch ermöglicht, wobei der Austausch ohne eine nachfolgende sicherheitstechnische Kontrolle erfolgen kann. Ferner besteht die Aufgabe der Erfindung darin, ein Set mit einem solchen Buchseneinsatz und einem Entnahmewerkzeug anzugeben. Eine weitere Aufgabe der Erfindung besteht darin, ein Verfahren zum Entnehmen eines solchen Buchseneinsatzes anzugeben.

Erfindungsgemäß wird diese Aufgabe im Hinblick auf das elektrochirurgische Gerät durch den Gegenstand des Patentanspruchs 1, im Hinblick auf das Set durch den Gegenstand des Patentanspruchs 10 und im Hinblick auf das Entnahmeverfahren durch den Gegenstand des Patentanspruchs 13 gelöst.

So beruht die Erfindung auf dem Gedanken, ein elektrochirurgisches Gerät mit einem Buchseneinsatz anzugeben, der eine Frontplatte umfasst, die wenigstens eine Steckeröffnung aufweist. Der Buchseneinsatz weist ferner erste und zweite Seitenwände auf, die mit der Frontplatte verbunden sind und einen Aufnahmeraum für elektronische Bauteile begrenzen. Außerdem weist der Buchseneinsatz wenigstens ein Rastmittel auf, das mit der ersten Seitenwand verbunden und von einer Arretierstellung in eine Entriegelungsstellung überführbar ist. Wenigstens die erste Seitenwand weist einen Führungskanal auf, der eine Zugangsöffnung in der Frontplatte bildet und durch den das Rastmittel zum Entriegeln betätigbar ist.

Die der Erfindung zugrundeliegende Idee besteht darin, einen Buchseneinsatz derart zu gestalten, dass die Entnahme des Buchseneinsatzes möglich ist, ohne das Gehäuse des elektrochirurgischen Gerätes öffnen zu müssen. So kann ein Austausch des Buchseneinsatzes erfolgen und gleichzeitig eine Manipulation im Inneren des elektrochirurgischen Gerätes vermieden werden. Dazu ist der Führungskanal vorgesehen, der in der Frontplatte eine Zugangsöffnung bildet. Der Führungskanal ermöglicht so den Zugriff auf ein Rastmittel, das zum Entriegeln des Buchseneinsatzes dient. Insbesondere kann wenigstens in der ersten Seitenwand ein Führungskanal für ein Betätigungselement ausgebildet sein, wobei das Rastmittel zum Entriegeln durch das Betätigungselement betätigbar ist. Der Führungskanal ermöglicht also einen Zugang für das Betätigungselement zum Rastmittel. Da der Führungskanal in der Frontplatte eine Zugangsöffnung bildet, ist der im Inneren des Buchseneinsatzes verborgene Rastmechanismus einfach von außen zugänglich. Dies erleichtert die Entnahme des Buchseneinsatzes und vermeidet eine aufwändige Demontage des elektrochirurgischen Geräts.

Bei dem erfindungsgemäßen elektrochirurgischen Gerät ist im Führungskanal des Buchseneinsatzes eine axial verschiebbare Betätigungsstange angeordnet. Dabei ist ein erstes Ende der Betätigungsstange zum Entriegeln des Rastmittels ausgebildet. Vorzugsweise ist das erste Ende der Betätigungsstange, insbesondere zum Entriegeln des Rastmittels, in Längsrichtung des Führungskanals beweglich angeordnet. Bei der Betätigung des Rastmittels verlässt das erste Ende der Betätigungsstange den Führungskanal in Richtung des Innenraums des Geräts. Somit ist das Betätigungsmittel aus dem Führungskanal herausbewegbar angeordnet. Im Allgemeinen ist vorgesehen, ein Betätigungselement im Führungskanal axial verschiebbar zu führen, um den Rastmechanismus zu betätigen, d.h. das Rastmittel in die Entriegelungsstellung zu überführen. Das Betätigungselement kann beispielsweise ein Werkzeug mit einer entsprechend langen Entriegelungsstange sein, das den Führungskanal vollständig durchgreift und sich so von der Frontplatte bis zum Rastmittel erstreckt. Besonders bevorzugt ist es, wenn im Führungskanal eine Betätigungsstange angeordnet ist. Die Betätigungsstange kann ein erstes Ende, das auf das Rastmittel wirkt, und ein zweites Ende aufweisen, das in der Arretierstellung bündig mit der Zugangsöffnung in der Frontplatte abschließt. Damit ist sichergestellt, dass der Führungskanal permanent verschlossen ist, was aus hygienischer und ästhetischer Sicht vorteilhaft ist.

Die Länge der Betätigungsstange kann größer als die Länge des Führungskanals sein. Vorzugsweise ist das Rastmittel fluchtend mit dem Führungskanal an der ersten Seitenwand angeordnet. Das Rastmittel kann unmittelbar an einer rückwärtigen Austrittsöffnung des Führungskanals oder im Abstand zu dieser angeordnet sein. Die Position des Rastmittels, der Abstand zur rückwärtigen Austrittsöffnung des Führungskanals, hat einen Einfluss auf die Belastung des vorzugsweise aus Kunststoff gebildeten Rastmittels. Wenn das erste Ende der Betätigungsstange unmittelbar an dem Rastmittel anliegt, wird die Weglänge für die Betätigung des Rastmittels reduziert. Dadurch ist zur Betätigung des Rastmittels nur ein kurzer Hub erforderlich.

Die Betätigungsstange bzw. das erste Ende der Betätigungsstange kann ferner einen Anschlag aufweisen, der mit dem Führungskanal zur Begrenzung der Axialbewegung der Betätigungsstange zusammenwirkt. Der Anschlag kann insbesondere mit der rückwärtigen Austrittsöffnung des Führungskanals zusammenwirken. Durch den Anschlag an der Betätigungsstange wird sichergestellt, dass die Betätigungsstange in dem Buchseneinsatz verbleibt. Der Anschlag bildet insofern eine Verliersicherung.

In einer weiteren bevorzugten Ausführungsform der vorliegenden Erfindung weist das Rastmittel einen keilförmigen Vorsprung auf, der mit der Betätigungsstange fluchtet und dessen Höhe in Vorschubrichtung der Betätigungsstange zunimmt. Mit anderen Worten weist der Vorsprung eine Steigung auf, die mit steigendem Abstand zur rückwärtigen Austrittsöffnung des Führungskanals größer wird. Damit ist sichergestellt, dass durch den axialen Vorschub der Betätigungsstange das Rastmittel einfach in die Entriegelungsstellung überführt wird. Folglich gewährleistet dies eine einfache und sichere mechanische Entriegelung des Buchseneinsatzes.

Das Rastmittel kann außerdem eine nach außen gerichtete Rastnase aufweisen, die zum Entriegeln nach innen in Richtung des Aufnahmeraumes bewegbar ist. Die Entriegelungsrichtung nach innen zum Aufnahmeraum hin ist im Hinblick auf das Gesamtsystem vorteilhaft, da auf diese Weise eine entsprechende Buchsenaufnahme für den Buchseneinsatz konstruktiv einfach gestaltet werden kann. Insgesamt wird auf diese Weise ein kompakter Aufbau sowohl des Buchseneinsatzes als auch des Gesamtsystems, insbesondere des chirurgischen Geräts, ermöglicht. Ferner wird auf diese Weise vermieden, dass der Rastmechanismus einen Lichtfpad eines optional den Buchseneinsatz ummantelnden Lichtleiters behindert.

In einer weiteren bevorzugten Ausführungsform des erfindungsgemäßen elektrochirurgischen Geräts weisen die erste Seitenwand und die zweite Seitenwand des Buchseneinsatzes jeweils einen Führungskanal auf. Insgesamt können also zwei Führungskanäle vorgesehen sein, wobei in jeder Seitenwand des Buchseneinsatzes jeweils ein Führungskanal angeordnet ist. Die Führungskanäle können zueinander parallel ausgerichtet sein. Insbesondere können die Führungskanäle auf gleicher Höhe angeordnet sein. Wenn in jeder Seitenwand ein Führungskanal angeordnet ist, wird ein Verkeilen des Buchseneinsatzes beim Entnehmen aus einem elektrochirurgischen Gerät, insbesondere einer Buchsenaufnahme eines elektrochirurgischen Geräts, vermieden. Dies erhöht die Bediensicherheit des erfindungsgemäßen Buchseneinsatzes.

Bei dem erfindungsgemäßen elektrochirurgischen Gerät kann die Frontplatte des Buchseneinsatzes in einer bevorzugten Ausgestaltung einen lichtdurchlässigen Rahmen, insbesondere einen Streurahmen, aufweisen. Der Rahmen bzw. Streurahmen kann mit einem Lichtleiter verbunden sein. Es ist auch möglich, dass der Rahmen und der Lichtleiter aus einem Teil, einteilig und/oder nahtlos gebildet ist. Im Allgemeinen kann der Buchseneinsatz einen Streurahmen aufweisen, der die Frontplatte umrandet. Der lichtdurchlässige Rahmen dient als optische Anzeige für den Betriebszustand des elektrochirurgischen Geräts. Indem diese Betriebszustandsanzeige in den Buchseneinsatz integriert ist, wird das optische Erscheinungsbild des gesamten elektrochirurgischen Geräts verbessert.

Der Lichtleiter kann insbesondere ein Einschubgehäuse bilden, das den Aufnahmeraum vollumfänglich umgibt und mit einer Lichtquelle verbindbar ist. Der Buchseneinsatz ist insofern modular aufgebaut, wobei die einzelnen Module beispielsweise durch den von den Seitenwänden und der Frontplatte begrenzten Aufnahmeraum, durch die Betätigungsstange und durch den Lichtleiter gebildet sein können. Im Allgemeinen erleichtert der modulare Aufbau des Buchseneinsatzes dessen Montage.

In einer weiteren bevorzugten Ausgestaltung der Erfindung weist das Einschubgehäuse wenigstens ein komplementäres Mittel zur Verbindung mit dem Rastmittel der Seitenwand auf. Das komplementäre Mittel kann an der Innenwand des Einschubgehäuses außerhalb des Lichtpfades angeordnet sein. Das komplementäre Mittel dient zur Fixierung des Buchseneinsatzes im Einschubgehäuse. Dabei verrastet das Rastmittel mit dem komplementären Mittel, so dass der Buchseneinsatz im Einschubgehäuse fixiert ist. Die Anordnung des komplementären Mittels ist so gewählt, dass das komplementäre Mittel den Lichtpfad innerhalb des Einschubgehäuses nicht beeinträchtigt. Damit ist sichergestellt, dass der durch das Einschubgehäuse bewirkte homogene Lichteffekt nicht durch das Rastmittel gestört wird.

Bei dem erfindungsgemäßen elektrochirurgischen Gerät kann beispielsweise das Einschubgehäuse des Buchseneinsatzes fest mit dem Gehäuse des elektrochirurgischen Geräts verbunden sein. Der Buchseneinsatz bzw. der durch die Frontplatte und die Seitenwände begrenzte Aufnahmeraum kann über das Einschubgehäuse mit dem Gehäuse des elektrochirurgischen Geräts rastverbunden sein.

Ein nebengeordneter Aspekt der Erfindung betrifft ein Set mit wenigstens einem zuvor beschriebenen Buchseneinsatz und einem Entnahmewerkzeug. Das Entnahmewerkzeug kann wenigstens eine Entriegelungsstange mit einem freien Ende aufweisen. Das Entnahmewerkzeug kann auch zwei bügelartig verbundene Entriegelungsstangen mit jeweils einem freien Ende aufweisen. Jedenfalls ist vorgesehen, dass das freie Ende in den Führungskanal einführbar ist. Dabei kann das freie Ende zum Entriegeln des Rastmittels vollständig durch den Führungskanal führbar sein und unmittelbar auf das Rastmittel einwirken. Alternativ kann das freie Ende mit der Betätigungsstange zusammenwirken, die innerhalb des Führungskanals axial verschiebbar gelagert ist. Die Betätigung des Rastmittels erfolgt dann über die Betätigungsstange, die durch das Entriegelungswerkzeug im Führungskanal verschoben wird.

Die Entriegelungsstange kann einen vorstehenden Rand am freien Ende aufweisen. Vorzugsweise ist der Durchmesser des freien Randes kleiner als der Durchmesser des Führungskanals. Dies erleichtert das Einführen der Entriegelungsstange in den Führungskanal.

Der Führungskanal kann ferner eine Ausnehmung aufweisen, die durch den vorstehenden Rand zum Entnehmen des Buchseneinsatzes aus dem Gerät hintergreifbar ist. Auf diese Weise kann das Entnahmewerkzeug im Führungskanal arretiert werden. Das Entnahmewerkzeug erfüllt so eine Doppelfunktion. Einerseits ermöglicht das Entnahmewerkzeug ein Verschieben der Betätigungsstange bzw. eine direkte Betätigung des Rastmittels. Andererseits bewirkt das Entnahmewerkzeug die Entnahme des Buchseneinsatzes, da das Entnahmewerkzeug mit dem Führungskanal in der Seitenwand des Buchseneinsatzes zumindest temporär formschlüssig verbindbar ist. Im Verbindungszustand bildet das Entnahmewerkzeug also einen Haltebügel, an welchem der Buchseneinsatz aus dem Einschubgehäuse gezogen werden kann.

Gemäß einem ergänzenden, nebengeordneten Aspekt wird im Rahmen der vorliegenden Anmeldung ein Verfahren zum Entnehmen des zuvor beschriebenen Buchseneinsatzes aus einem elektrochirurgischen Gerät offenbart und beansprucht, bei dem das Entnahmewerkzeug mit der Entriegelungsstange in den Führungskanal des Buchseneinsatzes eingeführt und der Buchseneinsatz entriegelt wird. Ferner wird bei dem erfindungsgemäßen Verfahren das Entnahmewerkzeug gekippt und mit dem Führungskanal verbunden. Der Buchseneinsatz wird mit dem Entnahmewerkzeug aus dem Gerät entnommen, insbesondere gezogen. Die Erfindung zeigt somit ein vergleichsweise einfaches Verfahren zum Entnehmen eines Buchseneinsatzes aus einem elektrochirurgischen Gerät auf, bei dem ein Öffnen des Geräts vermieden wird. Das erfindungsgemäße Verfahren kann daher auch von ungeschultem Personal durchgeführt werden und vermeidet bzw. erspart eine nachfolgende sicherheitstechnische Kontrolle.

Die Erfindung wird im Folgenden anhand von Ausführungsbeispielen unter Bezugnahme auf die beigefügten, schematischen Zeichnungen näher erläutert. Darin zeigen
- Figur 1: eine perspektivische Rückansicht eines erfindungsgemäßen Buchseneinsatzes nach einem bevorzugten Ausführungsbeispiel;
- Figur 2: eine perspektivische Vorderansicht des Buchseneinsatzes gemäß Figur 1;
- Figur 3: eine Explosionsdarstellung des Buchseneinsatzes gemäß Figur 1;
- Figur 4: eine Explosionsdarstellung eines Bereichs eines Gerätegehäuses eines elektrochirurgischen Geräts mit den Buchseneinsatz gemäß Figur 1;
- Figur 5: eine perspektivische Schnittansicht des Gerätegehäuses gemäß Figur 4 mit eingesetztem Buchseneinsatz;
- Figur 6: eine weitere perspektivische Schnittansicht des Gerätegehäuses gemäß Figur 4;
- Figur 7: eine Schnittansicht durch das Gerätegehäuse gemäß Figur 4 und den darin eingesetzten Buchseneinsatz;
- Figur 8: eine horizontale Schnittansicht durch das Gerätegehäuse gemäß Figur 4 und den Buchseneinsatz;
- Figur 9: eine perspektivische Ansicht eines Entnahmewerkzeugs für den erfindungsgemäßen Buchseneinsatz nach einem bevorzugten Ausführungsbeispiel;
- Figur 10: eine Draufsicht auf das Entnahmewerkzeug gemäß Figur 9 bei Verbindung mit dem Buchseneinsatz; und
- Figur 11: eine Detailansicht der Verbindung zwischen dem Entnahmewerkzeug und den Buchseneinsatz gemäß Figur 10.

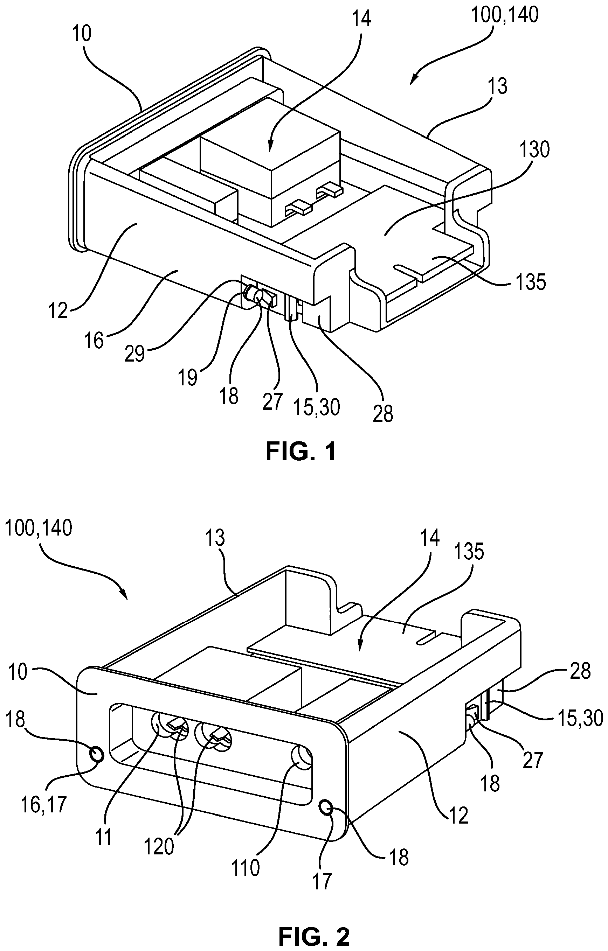

Figur 1 zeigt den Buchseneinsatz 100 mit einem Aufnahmeraum 14, der durch Seitenwände 12, 13 und eine Frontplatte 10 begrenzt ist. Der Buchseneinsatz 100 bildet einen Träger bzw. ein Gehäuse für ein oder mehrere Buchsen 110, 120, die über Steckeröffnungen 11 in der Frontplatte 10 zugänglich sind. Bei dem dargestellten Buchseneinsatz 100 handelt es sich vorzugsweise um einen Buchseneinsatz für ein elektrochirurgisches Gerät, insbesondere ein HF-Chirurgiegerät. Der Buchseneinsatz 100 weißt insgesamt drei Buchsen 110, 120 auf, wobei eine erste Buchse 110 und -zwei zweite Buchsen 120 vorgesehen sind. Die zweiten Buchsen 120 bilden ein Buchsenpaar. Ferner ist im Aufnahmeraum 14 eine Leiterplatte 130 angeordnet, die eine rückwärtige Steckverbindung 135 bildet, so dass die Buchsen 110, 120 des Buchseneinsatzes 100 über die Steckverbindung 135 der Leiterplatte 130 mit Komponenten eines elektrochirurgischen Geräts verbindbar sind.

Wie in Figur 1 ferner erkennbar ist, weist die erste Seitenwand 12 des Buchseneinsatzes 100 einen Führungskanal 16 auf, der senkrecht zur Frontplatte 10 angeordnet ist. Die Seitenwand 12 ist in einem spitzen Winkel zur Frontplatte 10 angeordnet, sodass die Weite des Buchseneinsatzes im Bereich der Frontplatte größer ist als in einem Bereich, der von der Frontplatte beabstandet angeordnet ist. Der Führungskanal 16 umfasst eine rückwärtige Austrittsöffnung 29, die in eine Aussparung 28 in der ersten Seitenwand 12 mündet. Eine entsprechende Gestaltung weist die zweite Seitenwand 13 auf. Insofern sind bei dem dargestellten Ausführungsbeispiel des Buchseneinsatzes 100 zwei Seitenwände 12,13 vorgesehen, die jeweils einen Führungskanal 16 aufweisen. Die Führungskanäle 16 umfassen jeweils eine rückwärtige Austrittsöffnung 29. Ferner sind die Seitenwände 12, 13 in einem rückwärtigen Bereich des Buchseneinsatzes 100 mit einer Aussparung 28 versehen, in der jeweils ein Rastmittel 15 angeordnet ist. In Figur 1 ist gut erkennbar, dass das Rastmittel 15 in Verlängerung der Längsachse des Führungskanals im Wesentlichen mit dem Führungskanal 16 fluchtend ausgebildet ist.

Das Rastmittel 15 weist einen keilförmigen Vorsprung 27 auf, der in einer Flucht zum Führungskanal 16 angeordnet ist. Beabstandet von dem keilförmigen Vorsprung 27 ist eine nach außen gerichtete Rastnase 30 vorgesehen. Die Rastnase 30 ist an einem freien Ende des Rastmittels 15 angeordnet. Es ist auch möglich, dass der keilförmige Vorsprung 27 direkt in die Rastnase 30 übergeht und zwischen beiden kein Abstand vorhanden ist (nicht dargestellt).

Durch den Führungskanal 16 erstreckt sich bei dem dargestellten Buchseneinsatz 100 eine Betätigungsstange 18. Die Betätigungsstange 18 weist eine Länge auf, die größer als die Länge des Führungskanals 16 ist. Insbesondere steht ein erstes Ende der Betätigungsstange 18 über die Austrittsöffnung 29 vor. Die Betätigungsstange 18 ist vorzugsweise fluchtend mit dem keilförmigen Vorsprung 27 des Rastmittels 15 ausgerichtet. An ihrem ersten Ende weist die Betätigungsstange 18 einen Anschlag 19 auf, der mit der Austrittsöffnung 29 zusammenwirkt, so dass eine Axialbewegung der Betätigungsstange 18 in Richtung zur Frontplatte 10 des Buchseneinsatzes 100 begrenzt ist. Der Anschlag 19 kann beispielsweise als ringförmige Rippe ausgebildet sein, die einen größeren Durchmesser als der Führungskanal 16 aufweist.

In Figur 2 ist gut erkennbar, dass die Führungskanäle 16 in der Frontplatte jeweils eine Zugangsöffnung 17 ausbilden. Die Zugangsöffnung 17 ist durch die Betätigungsstange 18 verschlossen. In der Arretierstellung des Buchseneinsatzes 100 bildet ein zweites Ende der Betätigungsstange 18 mit der Frontplatte 10 vorzugweise eine gemeinsame Ebene. Mit anderen Worten entspricht der Abstand zwischen einer vorderen Endfläche der Betätigungsstange 18 und dem Anschlag 19 der Länge des Führungskanals 16.

In Figur 2 ist außerdem erkennbar, dass die Frontplatte 10 einen versenkten Mittelteil aufweist, in dem die Steckeröffnungen 11 angeordnet sind. Die Steckeröffnungen 11 ermöglichen den Zugang zu den ersten und zweiten Buchsen 110, 120.

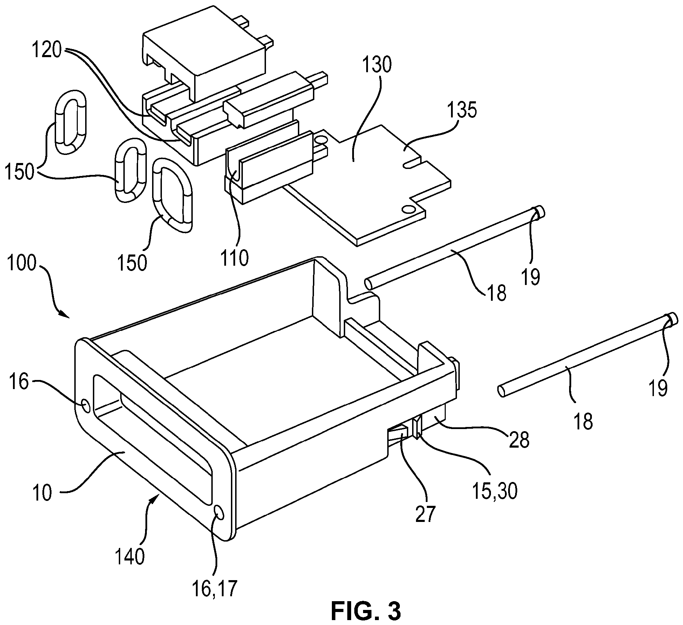

In der Explosionsdarstellung gemäß Figur 3 ist der innere Aufbau des Buchseneinsatzes 100 gut erkennbar. Der Buchseneinsatz 100 weist insbesondere ein Innengehäuse 140 auf, das vorzugsweise einstückig ausgebildet ist. Das Innengehäuse 140 kann als Spritzgussteil hergestellt sein. Das Innengehäuse 140 umfasst die Frontplatte 10 und die mit der Frontplatte 10 fest verbundenen bzw. einteilig ausgebildeten Seitenwände 12, 13. Das Innengehäuse 140 begrenzt so den Aufnahmeraum 14 für die elektronischen Bauteile, der insbesondere die einzelnen Buchsen 110, 120 sowie die Leiterplatte 130 aufnimmt. In Figur 3 sind ferner Federmittel 150 erkennbar, durch die die Stecköffnungen der einzelnen Buchsen 110, 120 variabel ausgebildet sind, sodass Pins mit unterschiedlichen Durchmessern kontaktsicher in die Buchsen 110, 120 gesteckt werden können.

Die Führungskanäle 16 sind ebenfalls einteilig mit dem Innengehäuse 110 bzw. dem Buchseneinsatz 100 ausgebildet. Die Führungskanäle 16 nehmen die Betätigungsstange 18 auf, die zum Entriegeln des Buchseneinsatzes 100 mit den Rastmitteln 15 zusammenwirken. Die Betätigungsstangen 18 bilden im Wesentlichen Stößel, die durch axiale Verschiebung auf das Rastmittel 15 einwirken, so dass die Rastnase 30 zum Entriegeln des Buchseneinsatz 100 nach innen ausgelegt wird. Das Rastmittel 15 bildet im Wesentlichen einen Schnapphaken, der durch Betätigung der Betätigungsstange 18 in die Entriegelungsstellung gebracht werden kann. Dabei wird die Betätigungsstange 18 gegen den keilförmigen Vorsprung 27 geschoben, so dass das rückwärtige, erste Ende der Betätigungsstange 18 entlang der schiefen Ebene des keilförmigen Vorsprungs 27 gleitet. Dies bewirkt eine nach innen gerichtete Auslenkung des Rastmittel 15.

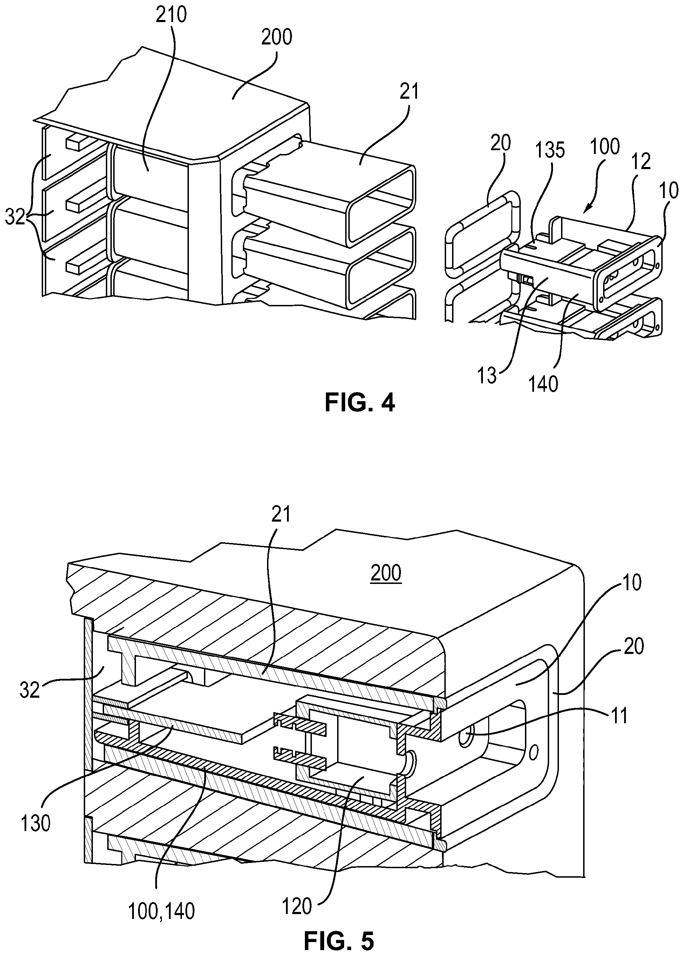

Der Buchseneinsatz 100 ist mit einen Einschubgehäuse 21 verbindbar, das wiederum fest in einem Gerätegehäuse 200 eines elektrochirurgischen Geräts angeordnet sein kann (Figur 4). Das Einschubgehäuse 21 umschließt den Aufnahmeraum 14 vorzugweise vollständig, so dass die darin angeordneten elektronischen Bauteile vor Zugriff geschützt sind. Das Einschubgehäuse 21 kann aus einem transparenten Kunststoff hergestellt sein, beispielsweise durch Spritzgießen. Im Allgemeinen bildet das Einschubgehäuse 21 vorzugsweise einen Lichtleiter, der optisch mit einem lichtdurchlässigen Rahmen 20 gekoppelt sein kann, welcher im montierten Zustand des Buchseneinsatzes 100 die Frontplatte 10 umrahmt.

Das Einschubgehäuse 21 ist vorzugweise derart dimensioniert, dass die Frontplatte 10 im eingebauten Zustand des Buchseneinsatzes 100 mit einer Gehäusefront des elektrochirurgischen Geräts fluchtet. Der lichtdurchlässige Rahmen 20 kann über die Frontplatte 10 bzw. die Gehäusefront vorstehen. Dies ermöglicht einen dreidimensionalen Lichteffekt, der optisch ansprechend ist. Der über die Frontplatte 10 vorstehende Rahmen 20 ist gut in Figur 5 erkennbar.

Der lichtdurchlässige Rahmen 20 kann als Betriebszustandsanzeige genutzt werden, wobei Licht von einer Lichtquelle über das als Lichtleiter ausgebildete Einschubgehäuse 21 in den lichtdurchlässigen Rahmen 20 eingekoppelt wird. Die Lichtquelle wird vorzugsweise durch eine Hinterleuchtungsleiterplatte 32 gebildet, die hinter dem Buchseneinsatz 100 im Gerätegehäuse 200 angeordnet ist. In Figur 4 sind mehrere Hinterleuchtungsleiterplatten 32 gezeigt, die jeweils mehrere Leuchtdioden aufweisen. Die Leuchtdioden dienen als Lichtquellen für das als Lichtleiter ausgebildete Einschubgehäuse 21.

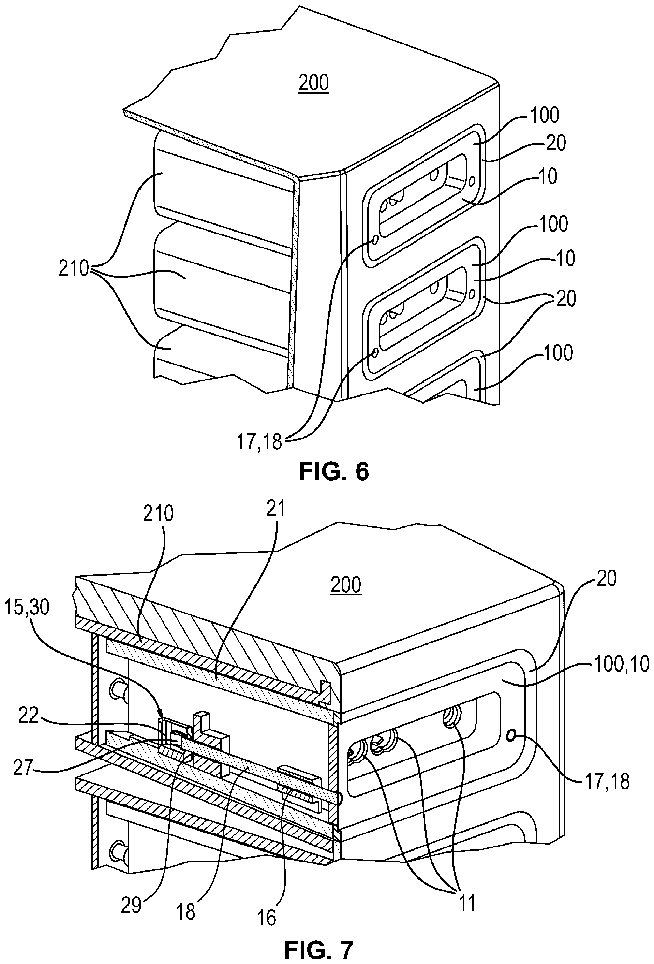

Wie in den Figuren 4-8 gut erkennbar ist, weist das Gerätegehäuse 200 ein oder mehrere Buchsenaufnahmen 210 auf, die derart dimensioniert sind, dass sie den Buchseneinsatz 100, insbesondere das Einschubgehäuse 21, aufnehmen. Das Einschubgehäuse 21 ist in der Buchsenaufnahme 210 fest arretiertbar. Das Einschubgehäuse 21 verbleibt vorzugsweise dauerhaft in der Buchsenaufnahme 210. Der Buchseneinsatz 100 ist hingegen in Einschubgehäuse 21 lösbar verrastet. Über den Rastmechanismus ist eine formschlüssige Verbindung zwischen dem Buchseneinsatz 100 und dem Einschubgehäuse 21 sichergestellt. Mithilfe eines Entnahmewerkzeugs 23 kann der Buchseneinsatz 100 entriegelt und aus dem Einschubgehäuse 21 herausgezogen werden.

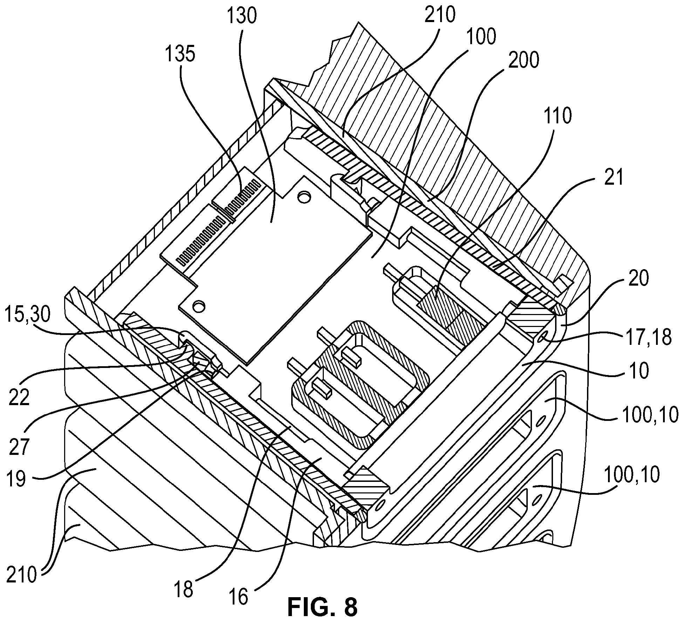

Das Rastmittel 15 zur Verbindung des Buchseneinsatzes 100 mit dem Einschubgehäuse 21 ist in Figur 8 gut zu sehen. Insbesondere ist in Figur 8 erkennbar, dass das Rastmittel 15 des Buchseneinsatzes 100 einem keilförmigen Vorsprung 27 und eine Rastnase 30 aufweist. Die Rastnase 30 hintergreift in der Arretierstellung ein komplementäres Mittel 22 des Einschubgehäuse 21, so dass eine formschlüssige Verbindung zwischen Buchseneinsatz 100 und Einschubgehäuse 21 hergestellt ist. Das komplementäre Mittel 22 ist durch einen nach innen ragenden Rastvorsprung gebildet, der einteilig mit dem Einschubgehäuse 21 ausgebildet ist.

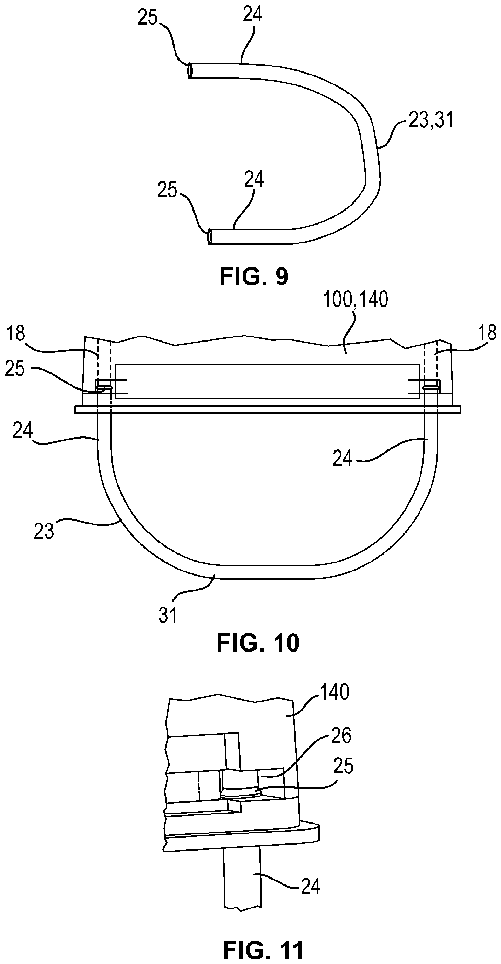

Zum Entriegeln des Buchseneinsatzes 100 kommt ein Entnahmewerkzeug 23 zum Einsatz, dass in Figur 9 gezeigt ist. Das Entnahmewerkzeug 23 umfasst zwei Entriegelungsstangen 24, die durch einen Bügel 31 miteinander verbunden sind. Der Bügel 31 und die Entriegelungsstangen 24 sind vorzugsweise einstückig miteinander ausgebildet. Die Entriegelungsstangen 24 weisen jeweils ein freies Ende auf, das mit einem vorstehenden Rand 25 versehen ist. Der vorstehende Rand 25 bildet im Wesentlichen einen ringförmigen Vorsprung am freien Ende der Entriegelungsstange 24. Der Durchmesser des vorstehenden Rands 25 ist vorzugsweise kleiner als der Durchmesser des Führungskanals 16, so dass das Entnahmewerkzeug 23, insbesondere die Entriegelungsstange 24, leicht in den Führungskanal 16 einführbar ist. Insbesondere kann das Entnahmewerkzeug 23 über die Zugangsöffnungen 17 in die Führungskanäle 16 eingeführt werden und gelangt somit in Kontakt mit der im Führungskanal 16 angeordneten Betätigungsstange 18. Durch das Entnahmewerkzeug 24 können also die Betätigungsstangen 18 axial im Führungskanal 16 verschoben werden. Dabei gelangt jeweils das rückwärtige, erste Ende der Betätigungsstange 18 in Kontakt mit dem keilförmigen Vorsprung 27 der Rastmittel 15. Durch das Einführen des Entnahmewerkzeugs 23 wird daher das Rastmittel 15 ausgelenkt und von der Arretierstellung in die Entriegelungsstellung überführt.

Wie in den Figuren 10 und 11 erkennbar ist, weist jeder Führungskanal 16 vorzugsweise eine Ausnehmung 26 auf, die nahe der Frontplatte 10 in der Seitenwand 12, 13 ausgebildet ist. Die Ausnehmung 26 ist derart ausgebildet, dass der vorstehende Rand 25 des Entnahmewerkzeugs 23 formschlüssig mit den Buchseneinsatz 100 verbindbar ist. Auf diese Weise kann das Entnahmewerkzeug 23 als Haltebügel genutzt werden, um den Buchseneinsatz 100 aus dem Gerätegehäuse 200 zu entfernen.

Zum Einbau des Buchseneinsatzes 100 wird dieser einfach in das Einschubgehäuse 21 eingeschoben, das fest in der Buchsenaufnahme 210 des Gerätegehäuses 200 angeordnet, insbesondere stoffschlüssig oder mechanisch verankert, ist. Beispielsweise kann das Einschubgehäuse 21 mit der Buchsenaufnahme 210 stoff- oder formschlüssig verbunden sein. Durch Hineinschieben des Buchseneinsatzes 100 verrastet das Rastmittel 15 mit dem komplementären Mittel 22 in dem Einschubgehäuse 21, so dass der Buchseneinsatz 100 im Gerätegehäuse 200 fixiert ist.

Da das Einschubgehäuse 21 gleichzeitig einen Lichtleiter bildet, ist der Rastmechanismus bzw. Schnapphakenmechanismus des Buchseneinsatzes 100 in den Aussparungen 28 der Seitenwände 12,13 angeordnet. So wird verhindert, dass der Rastmechanismus einem Lichtpfad im Lichtleiter behindert. Die elektrische Verbindung zwischen den Buchsen 110,120 und den elektrischen Komponenten im elektrochirurgischen Gerät wird über die Leiterplatte 130 hergestellt, die eine rückwärtige Steckverbindung 135 aufweist. Die elektrische Verbindung wird durch Einschieben des Buchseneinsatzes 100 in die Buchsenaufnahme 210 bereitgestellt, wobei die Steckerverbindung 135 mit einer entsprechenden Buchsenverbindung im Gerätegehäuse 200 in Eingriff gelangt.

Zum Wechseln des Buchseneinsatzes 100 wird das Entnahmewerkzeug 23 genutzt, das auf die Betätigungsstangen 18 bzw. Stößel wirkt, die in den Führungskanälen 16 angeordnet sind. Über die Betätigungsstangen 18 kann mit dem Entnahmewerkzeug die Rastnase 30 des Rastmittels 15 nach innen ausgelenkt werden, so dass der Buchseneinsatz 100 freigegeben wird. Durch das Einschieben des Entnahmewerkzeugs 23 in die Führungskanäle 16 verbindet sich das Entnahmewerkzeug 23 formschlüssig mit den Buchseneinsatz 100. Dazu ist die Ausnehmung 26 in den Führungskanälen 16 vorgesehen, in die der vorstehende Rand 25 der Entriegelungsstange 24 eingreift. Die Ausnehmung 26 kann etwa 3 mm bis 4 mm von der Frontplatte 10 beabstandet in dem Führungskanal 16 angeordnet sein. Um den Formschluss zwischen dem Entnahmewerkzeug 23 und dem Buchseneinsatz 100 herzustellen, wird das Entnahmewerkzeug 23 gekippt. Dadurch gelangt der vorstehende Rand 25 in einen formschlüssigen Eingriff mit der Ausnehmung 26. Anschließend kann mit Hilfe des Entnahmewerkzeugs 23 der Buchseneinsatz 100 aus dem Einschubgehäuse 21 gezogen werden.

Alternativ ist es möglich, anstelle der Betätigungsstange 18 ein Entnahmewerkzeug 23 einzusetzen, dessen Entriegelungsstangen 24 eine ausreichende Länge aufweisen, um direkt auf die Rastmittel 15 einzuwirken. Die Verwendung von Betätigungsstangen 18, die fest in die Führungskanäle 16 integriert sind, hat jedoch Vorteile, da der Buchseneinsatz 100 auf diese Weise vollständig abgeschlossen ist. Durch die Betätigungsstangen 18, die in der Arretierstellung bündig mit der Frontplatte 10 abschließen, vermittelt der Buchseneinsatz 100 einen optisch ansprechenden Gesamteindruck.

Beim Vorschieben der Betätigungsstange 18 verrastet der Anschlag 19 der Betätigungsstange 18 außerdem mit dem keilförmigen Vorsprung 27, so dass die Betätigungsstange 18 in der Entriegelungsstellung arretiert wird. Der Anschlag 19 weist insofern eine Doppelfunktion auf. Einerseits verhindert der Anschlag 19, dass die Betätigungsstange 18 im Arretierzustand nach vorne, also über die Zugangsöffnung 17, aus dem Führungskanal 16 entnommen werden kann. Gleichzeitig verhindert der Anschlag 19 in der Entriegelungsstellung, dass die Betätigungsstange 18 in die Arretierstellung zurückfällt. So kann verhindert werden, dass ein bereits aus der Buchsenaufnahme 110 entnommener Buchseneinsatz 100 wieder in die Buchsenaufnahme 210 eingesetzt wird. Dies ist erst möglich, wenn die Betätigungsstange 18 manuell in ihre Ausgangslage, also die Arretierstellung zurückgebracht wurde.

Mit der vorliegenden Erfindung kann ein Austausch des Buchseneinsatzes 100 erfolgen, ohne das Gerätegehäuse 200 des elektrochirurgischen Geräts öffnen zu müssen. Ein Zugriff auf stromführende Bauteile des elektrochirurgischen Geräts während des Austauschs des Buchseneinsatzes 100 wird so vermieden. Daher ist nach dem Austausch des Buchseneinsatzes 100 keine zusätzliche sicherheitstechnische Kontrolle erforderlich. Dies reduziert den Wartungsaufwand für elektrochirurgische Geräte. Außerdem ist der Austausch des Buchseneinsatzes 100 vereinfacht, da dazu keine aufwändigen Werkzeuge eingesetzt werden müssen. Insbesondere sind keine Schaubverbindungen oder dergleichen zu lösen, um den Buchseneinsatz 100 auszutauschen. Vielmehr erfolgt der Austausch des Buchseneinsatzes 100 einfach und schnell mithilfe des Entnahmewerkzeugs 23.

### Bezugszeichenliste

- 100: Buchseneinsatz
- 110: erste Buchse
- 120: zweite Buchse
- 130: Leiterplatte
- 135: Steckverbindung
- 140: Innengehäuse
- 150: Federmittel
- 200: Gerätegehäuse
- 210: Buchsenaufnahme
- 10: Frontplatte
- 11: Steckeröffnung
- 12: erste Seitenwand
- 13: zweite Seitenwand
- 14: Aufnahmeraum
- 15: Rastmittel
- 16: Führungskanal
- 17: Zugangsöffnung
- 18: Betätigungsstange
- 19: Anschlag
- 20: lichtdurchlässiger Rahmen
- 21: Einschubgehäuse
- 22: komplementäres Mittel
- 23: Entnahmewerkzeug
- 24: Entriegelungsstange
- 25: vorstehender Rand
- 26: Ausnehmung
- 27: keilförmiger Vorsprung
- 28: Aussparung
- 29: Austrittsöffnung
- 30: Rastnase
- 31: Bügel
- 32: Hinterleuchtungsleiterplatte

## Patentansprüche

1. Elektrochirurgisches Gerät mit einem Buchseneinsatz für ein elektrochirurgisches Gerät, wobei der Buchseneinsatz aufweist:
- eine Frontplatte (10), die wenigstens eine Steckeröffnung (11) aufweist,
- erste und zweite Seitenwände (12, 13), die mit der Frontplatte (10) verbunden sind und einen Aufnahmeraum (14) für elektronische Bauteile begrenzen, und
- wenigstens ein Rastmittel (15), das mit der ersten Seitenwand (12) verbunden und von einer Arretierstellung in eine Entriegelungsstellung überführbar ist,
wobei wenigstens die erste Seitenwand (12) einen Führungskanal (16) aufweist, der eine Zugangsöffnung (17) in der Frontplatte (10) bildet und durch den das Rastmittel (15) zum Entriegeln betätigbar ist,
wobei eine axial verschiebbare Betätigungsstange (18) im Führungskanal (16) angeordnet ist, und wobei ein erstes Ende der Betätigungsstange (18) zum Entriegeln des Rastmittels (15) ausgebildet und aus dem Führungskanal (16) herausbewegbar ist.

2. Elektrochirurgisches Gerät nach Anspruch 1,
**dadurch gekennzeichnet, dass**
die Länge der Betätigungsstange (18) größer als die Länge des Führungskanals (16) ist.

3. Elektrochirurgisches Gerät nach Anspruch 1 oder 2,
**dadurch gekennzeichnet, dass**
die Betätigungsstange (18), insbesondere das erste Ende der Betätigungsstange (18), einen Anschlag (19) aufweist, der mit dem Führungskanal (16) zur Begrenzung der Axialbewegung der Betätigungsstange (18) zusammenwirkt.

4. Elektrochirurgisches Gerät nach einem der Ansprüche 1 bis 3,
**dadurch gekennzeichnet, dass**
das Rastmittel (15) einen keilförmigen Vorsprung (27) aufweist, der mit der Betätigungsstange (18) fluchtet wobei die Höhe des keilförmigen Vorsprungs (27) in Vorschubrichtung der Betätigungsstange (18) zunimmt.

5. Elektrochirurgisches Gerät nach einem der Ansprüche 1 bis 4,
**dadurch gekennzeichnet, dass**
das Rastmittel (15) eine nach außen gerichtete Rastnase aufweist, die zum Entriegeln nach innen in Richtung des Aufnahmeraumes (14) bewegbar ist.

6. Elektrochirurgisches Gerät nach einem der Ansprüche 1 bis 5,
**dadurch gekennzeichnet, dass**
die erste und zweite Seitenwand (12, 13) jeweils einen Führungskanal (16) aufweisen.

7. Elektrochirurgisches Gerät nach einem der Ansprüche 1 bis 6,
**dadurch gekennzeichnet, dass**
die Frontplatte (10) einen lichtdurchlässigen Rahmen (20), insbesondere einen Streurahmen, aufweist, der mit einem Lichtleiter verbunden ist.

8. Elektrochirurgisches Gerät nach Anspruch 7,
**dadurch gekennzeichnet, dass**
der Lichtleiter ein Einschubgehäuse (21) bildet, das den Aufnahmeraum (14) vollumfänglich umgibt und mit einer Lichtquelle verbindbar ist.

9. Elektrochirurgisches Gerät nach Anspruch 8,
**dadurch gekennzeichnet, dass**
das Einschubgehäuse (21) wenigstens ein komplementäres Mittel (22) zur Verbindung mit dem Rastmittel (15) der Seitenwand (12, 13) aufweist, wobei das komplementäre Mittel (22) an der Innenwand des Einschubgehäuses (21) außerhalb des Lichtpfades angeordnet ist.

10. Set mit wenigstens einem Buchseneinsatz (100) eines elektrochirurgischen Geräts nach einem der vorhergehenden Ansprüche und einem Entnahmewerkzeug (23), das wenigstens eine Entriegelungsstange (24) mit einem freien Ende, insbesondere zwei bügelartig verbundene Entriegelungsstangen (24) mit jeweils einem freien Ende, aufweist, das in den Führungskanal (16) einführbar ist.

11. Set nach Anspruch 10,
**dadurch gekennzeichnet, dass**
die Entriegelungsstange (24) einen vorstehenden Rand (25) am freien Ende aufweist, wobei der Durchmesser des Randes (25) kleiner als der Durchmesser des Führungskanals (16) ist.

12. Set nach Anspruch 11,
**dadurch gekennzeichnet, dass**
der Führungskanal (16) eine Ausnehmung (26) aufweist, die durch den vorstehenden Rand (25) des Entnahmewerkzeugs (23) zum Entnehmen des Buchseneinsatzes aus dem Gerät hintergreifbar ist.

13. Verfahren zum Entnehmen eines Buchseneinsatzes aus einem elektrochirurgischen Gerät nach einem der Ansprüche 1 bis 9, bei dem
- das Entnahmewerkzeug (23) mit der Entriegelungsstange (24) in den Führungskanal (16) des Buchseneinsatzes eingeführt und der Buchseneinsatz entriegelt wird,
- das Entnahmewerkzeug (23) gekippt und mit einer Ausnehmung 26 des Führungskanals (16) formschlüssig verbunden wird, und
- der Buchseneinsatz mit dem Entnahmewerkzeug (23) aus dem Gerät entnommen wird.

## Claims

1. Electrosurgical device with a socket insert for an electrosurgical device, wherein the socket insert comprises:
- a front plate (10), which has at least one plug opening (11),
- first and second side walls (12, 13), which are connected to the front plate (10) and bound a receiving space (14) for electronic components, and
- at least one latching means (15), which is connected to the first side wall (12) and can be transferred from a locking position into an unlocking position,
wherein at least the first side wall (12) has a guide channel (16), which forms an access opening (17) in the front plate (10) and through which the latching means (15) can be actuated for unlocking,
wherein an axially displaceable actuating rod (18) is arranged in the guide channel (16), and wherein a first end of the actuating rod (18) is designed for unlocking the latching means (15) and can be moved out of the guide channel (16).

2. Electrosurgical device according to Claim 1,
**characterized in that**
the length of the actuating rod (18) is greater than the length of the guide channel (16).

3. Electrosurgical device according to Claim 1 or 2,
**characterized in that**
the actuating rod (18), in particular the first end of the actuating rod (18), has a stop (19), which interacts with the guide channel (16) for limiting the axial movement of the actuating rod (18).

4. Electrosurgical device according to one of Claims 1 to 3,
**characterized in that**
the latching means (15) has a wedge-shaped projection (27), which is in line with the actuating rod (18), wherein the height of the wedge-shaped projection (27) increases in the direction of advancement of the actuating rod (18).

5. Electrosurgical device according to one of Claims 1 to 4,
**characterized in that**
the latching means (15) has an outwardly directed latching lug, which for unlocking can be moved inwards in the direction of the receiving space (14).

6. Electrosurgical device according to one of Claims 1 to 5,
**characterized in that**
the first and second side walls (12, 13) respectively have a guide channel (16).

7. Electrosurgical device according to one of Claims 1 to 6,
**characterized in that**
the front plate (10) has a translucent frame (20), in particular a scattering frame, which is connected to a light guide.

8. Electrosurgical device according to Claim 7, **characterized in that**
the light guide forms a slide-in housing (21), which completely surrounds the receiving space (14) and can be connected to a light source.

9. Electrosurgical device according to Claim 8,
**characterized in that**
the slide-in housing (21) has at least one complementary means (22) for connecting to the latching means (15) of the side wall (12, 13), wherein the complementary means (22) is arranged on the inner wall of the slide-in housing (21) outside the light path.

10. Set with at least one socket insert (100) of an electrosurgical device according to one of the preceding claims and a removal tool (23), which has at least one unlocking rod (24) with a free end, in particular two unlocking rods (24) connected in a bow-like manner, with in each case a free end, which can be introduced into the guide channel (16).

11. Set according to Claim 10,
**characterized in that**
the unlocking rod (24) has a protruding edge (25) at the free end, wherein the diameter of the edge (25) is smaller than the diameter of the guide channel (16).

12. Set according to Claim 11,
**characterized in that**
the guide channel (16) has a recess (26), which can be gripped from behind by the protruding edge (25) of the removal tool (23) for the removal of the socket insert from the device.

13. Method for removing a socket insert from an electrosurgical device according to one of Claims 1 to 9, in which
- the removal tool (23) with the unlocking rod (24) is introduced into the guide channel (16) of the socket insert and the socket insert is unlocked,
- the removal tool (23) is tilted and brought into interlocking connection with a recess (26) of the guide channel (16), and
- the socket insert is removed from the device by the removal tool (23).

## Revendications

1. Dispositif électrochirurgical muni d'un insert de douille destiné à un dispositif électrochirurgical, l'insert de douille comportant :
- une plaque avant (10) qui comporte au moins une ouverture d'enfichage (11),
- des première et deuxième parois latérales (12, 13) qui sont reliées à la plaque avant (10) et qui délimitent un espace de réception (14) destiné à des composants électroniques, et
- au moins un moyen d'encliquetage (15) qui est relié à la première paroi latérale (12) et qui peut être transféré d'une position de verrouillage à une position de déverrouillage,
au moins la première paroi latérale (12) comportant un canal de guidage (16) qui forme une ouverture d'accès (17) dans la plaque avant (10) et à travers laquelle le moyen d'encliquetage (15) peut être actionné pour effectuer le déverrouillage,
une tige d'actionnement (18) déplaçable axialement étant disposée dans le canal de guidage (16) et une première extrémité de la tige d'actionnement (18) étant conçue pour déverrouiller le moyen d'encliquetage (15) et pouvant être sorti du canal de guidage (16).

2. Dispositif électrochirurgical selon la revendication 1,
**caractérisé en ce que**
la longueur de la tige d'actionnement (18) est supérieure à la longueur du canal de guidage (16).

3. Dispositif électrochirurgical selon la revendication 1 ou 2,
**caractérisé en ce que**
la tige d'actionnement (18), en particulier la première extrémité de la tige d'actionnement (18), comporte une butée (19) qui coopère avec le canal de guidage (16) pour limiter le mouvement axial de la tige d'actionnement (18).

4. Dispositif électrochirurgical selon l'une des revendications 1 à 3,
**caractérisé en ce que**
le moyen d'encliquetage (15) comporte une saillie (27) en forme de coin qui est alignée avec la tige d'actionnement (18), la hauteur de la saillie (27) en forme de coin augmentant dans le sens d'avancement de la tige d'actionnement (18).

5. Dispositif électrochirurgical selon l'une des revendications 1 à 4,
**caractérisé en ce que**
le moyen d'encliquetage (15) comporte un ergot d'encliquetage, orienté vers l'extérieur, qui peut être déplacé vers l'intérieur en direction de l'espace de réception (14) pour effectuer le déverrouillage.

6. Dispositif électrochirurgical selon l'une des revendications 1 à 5,
**caractérisé en ce que**
les première et deuxième parois latérales (12, 13) comportent chacune un canal de guidage (16).

7. Dispositif électrochirurgical selon l'une des revendications 1 à 6,
**caractérisé en ce que**
la plaque avant (10) comporte un cadre translucide (20), en particulier un cadre diffusant, qui est relié à un guide de lumière.

8. Dispositif électrochirurgical selon la revendication 7,
**caractérisé en ce que**
le guide de lumière forme un boîtier insérable (21) qui entoure complètement l'espace de réception (14) et qui peut être relié à une source de lumière.

9. Dispositif électrochirurgical selon la revendication 8,
**caractérisé en ce que**
le boîtier insérable (21) comporte au moins un moyen complémentaire (22) destiné à être relié au moyen d'encliquetage (15) de la paroi latérale (12, 13), le moyen complémentaire (22) étant disposé sur la paroi intérieure du boîtier coulissant (21) à l'extérieur du trajet lumineux.

10. Ensemble comprenant au moins un insert de douille (100) d'un dispositif électrochirurgical selon l'une des revendications précédentes et un outil d'extraction (23), qui comporte au moins une tige de déverrouillage (24) pourvue d'une extrémité libre, en particulier deux tiges de déverrouillage (24) reliées en forme d'étrier et pourvues chacune d'une extrémité libre, qui peut être introduite dans le canal de guidage (16).

11. Ensemble selon la revendication 10,
**caractérisé en ce que**
la tige de déverrouillage (24) comporte un bord saillant (25) à l'extrémité libre, le diamètre du bord (25) étant inférieur au diamètre du canal de guidage (16) .

12. Ensemble selon la revendication 11,
**caractérisé en ce que**
le canal de guidage (16) comporte un évidement (26) qui peut s'engager depuis l'arrière avec le bord saillant (25) de l'outil de retrait (23) pour retirer l'insert de douille du dispositif.

13. Procédé de retrait d'un insert de douille d'un dispositif électrochirurgical selon l'une des revendications 1 à 9, procédé dans lequel
- l'outil d'extraction (23) pourvu de la tige de déverrouillage (24) est inséré dans le canal de guidage (16) de l'insert de douille et l'insert de douille est déverrouillé,
- l'outil de retrait (23) est incliné et relié par complémentarité de formes à un évidement (26) du canal de guidage (16), et
- l'insert de douille est retiré du dispositif avec l'outil de retrait (23).
